# EUROPÄISCHE PATENTSCHRIFT

(11) **EP 0 928 311 B1**
(45) Veröffentlichungstag und Bekanntmachung des Hinweises auf die Patenterteilung: **09.04.2003**
(21) Anmeldenummer: 97943867.8
(22) Anmeldetag: 18.09.1997
(51) Int. Cl.: C08J 7/12, C08B 15/06

(54) **FORMGEGENSTAND MIT REAKTIVEN FUNKTIONEN**
SHAPING OBJECT WITH REACTIVE FUNCTIONS
OBJET FACONNE A FONCTIONS REACTIVES

(30) Priorität: 18.09.1996 DE 19638085
(43) Veröffentlichungstag der Anmeldung: 14.07.1999
(73) Patentinhaber: Gesellschaft fur Biotechnologische Forshung mbH, 38124 Braunschweig (DE)
(72) Erfinder: FRANK, Ronald, D-38124 Braunschweig (DE); MATYSIAK, Stefan, D-38124 Braunschweig (DE)
(74) Vertreter: Boeters, Hans Dietrich, Dr.
(86) Internationale Anmeldenummer: EP9705123
(87) Internationale Veröffentlichungsnummer: WO98012246

(56) Entgegenhaltungen:
- EP-A- 0 052 365
- EP-A- 0 339 502
- EP-A- 0 527 236
- US-A- 4 330 440
- US-A- 4 568 391
- PATENT ABSTRACTS OF JAPAN vol. 096, no. 008, 30.August 1996 & JP 08 100001 A (TEIJIN LTD), 16.April 1996,
- PATENT ABSTRACTS OF JAPAN vol. 016, no. 496 (C-0995), 14.Oktober 1992 & JP 04 183392 A (FUJI PHOTO FILM CO LTD), 30.Juni 1992,

## Beschreibung

Die vorliegende Erfindung betrifft einen Formgegenstand, dessen Oberfläche für chemische, biochemische oder biologische Anwendungen mit reaktiven Funktionen versehen ist.

Viele Polymermaterialien auf Basis natürlicher Polymere wie Polysaccharide (beispielsweise Zellulose, Stärke oder Chitin) oder organisch-synthetische Polymere wie Kunststoffe (beispielsweise Polyethylen, Polypropylen, Polytetrafluoroethylen oder Polystyrol) zeichnen sich durch Unlöslichkeit in organischen und wässrigen Medien aus. Deren Oberflächeneigenschaften wie Ladung, chemische Funktionalität oder Hydrophobizität wird für Synthese-, Trenn- und Nachweisreaktionen seit vielen Jahrzehnten beispielsweise auf folgenden Gebieten eingesetzt: chemische Festphasensynthese; hydrophobe Chromatographie, lonenaustausch-Chromatographie oder Affinitäts-Chromatographie bzw. Affinitätsanreicherung. In der klassischen Form als Granulate, Perlen oder Fasern werden diese Materialien in Reaktionsgefäßen wie Säulen oder Kartuschen geschüttet eingesetzt. Je nach Anwendung werden hierfür spezielle reaktive Funktionen auf der Oberfläche vorteilhaft ausgenutzt, wie sie beispielhaft durch die folgende schematische Darstellung belegt werden.

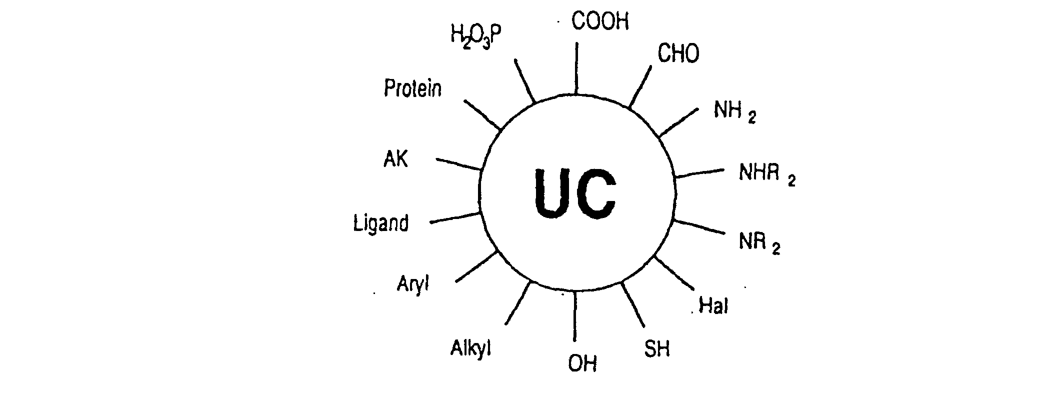

Solche Oberflächenfunktionalitäten können
a) direkt durch Co-Polymerisation mit entsprechenden Monomeren oder
b) nachträglich durch chemische Derivatisierung eingeführt werden.
Viele chemische, biochemische und biologische Arbeitstechniken nutzen speziell geformte Arbeitsgeräte (Formgegenstände wie Membranen, Folien, Papiere, miniaturisierte Reaktionsgefäße (von z. B. Eppendorf), Pipettenspitzen oder Mikrotiterplatten), die aus denselben oder gleichen Polymermaterialien hergestellt werden können, wie sie eingangs angesprochen wurden. Solche Formgegenstände erhalten durch einen optimierten Herstellungsprozeß eine für die Anwendung notwendige mechanische und chemische Stabilität. Die Oberflächen solcher Formgegenstände können ebenso, wie oben angegeben, vorteilhaft für gleiche Synthese-, Trenn- und Nachweisreaktionen eingesetzt werden. Die Einführung der verschiedenen für die Anwendung benötigten chemischen Funktionen ist hier jedoch problematischer, da durch den Derivatisierungsprozeß die mechanischen und/oder chemischen Eigenschaften nicht ungünstig beeinflußt werden dürfen.

Beispielsweise sind Formgegenstände wie Flachmaterial, beispielsweise Papier aus speziell derivatisierter Zellulose, beispielsweise Carboxymethyl-, Diethylaminoethyl-, Phospho-, Alkyl- oder Arylzellulose von großer Bedeutung. Die Herstellungsprozesse für Ausgangsmaterialien der genannten Formgegenstände erfordern jedoch hochgiftige Reagenzien, wie Bromcyan, Epichlorhydrin oder Acrylnitril, und/oder recht drastische alkalische Reaktionsbedingungen, so daß die daraus gefertigten Zellulosepapiere eine sehr viel geringere mechanische und chemische Stabilität aufweisen.

Die angesprochenen Probleme werden auch durch WO-A-94/19694 = US 5 576 220 noch nicht in vollem Umfang gelöst.

Nach US 4 568 391 wird Papier vorzugsweise als Papierpulpe mit Oberflächenvergütungsmitteln in einer einstufigen Reaktion umgesetzt, um die Papierqualität zu erhöhen.

Nach EP 0 339 502 werden Formgegenstände aus unter anderem regenerierter Zellulose in einer einstufigen Reaktion modifiziert. Formgegenstände aus nicht-regenerierter Zellulose oder aus Papier werden nicht angesprochen.

Aufgabe der vorliegenden Erfindung ist es nun, neue Materialien für die Biowissenschaften bzw. neue Formgegenstände vorzusehen, deren Oberfläche für chemische, biochemische oder biologische Anwendungen mit reaktiven Funktionen versehen sind, wobei diese Materialien insbesondere durch besonders schonende Verfahren vorgesehen werden sollen. Dadurch sollen die mechanischen und chemischen Eigenschaften der Formgegenstände nicht oder unwesentlich verändert werden.

Gemäß einer Ausführungsform wird die Erfindung zugrundeliegende Aufgabe durch einen Formgegenstand gelöst, dessen Oberfläche für chemische, biochemische oder biologische Anwendungen mit reaktiven Funktionen versehen ist und der dadurch herstellbar ist, daß man
(a) von einem geformten Polymermaterial auf Papierbasis als Formgegenstand ausgeht, dessen Oberfläche mit Hydroxylgruppen versehen ist, und den Formgegenstand in an sich bekannter Weise mit einem Carbonylierungsreagenz umsetzt und
(b) den carbonylierten Formgegenstand mit einer Verbindung der Formel R₃-NH-R₄, worin (i) R₃ den Rest eines an sich bekannten primären Amins und R₄ ein H-Atom oder (ii) R₃ und R₄, die gleich oder verschieden sein können, Reste eines an sich bekannten sekundären Amins darstellen, zu einem Urethan-Derivat umsetzt. Insbesondere kann R₃R₄NH ein Amin sein, das üblicherweise zur Urethan-Herstellung verwendet wird und als Urethanbildungsmittel bezeichnet werden kann.

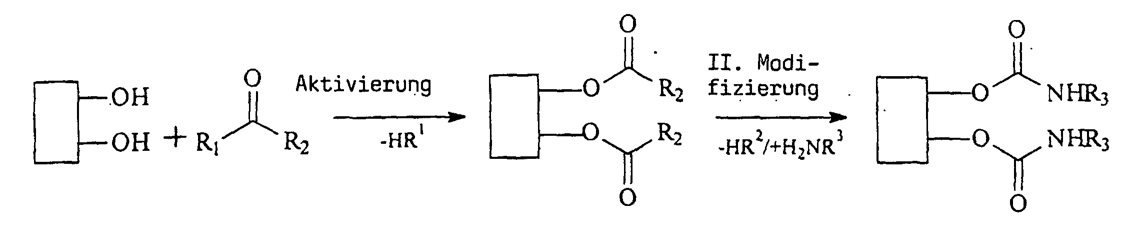

Die Umsetzung eines Polymeren, dessen Oberfläche mit Hydroxylgruppen versehen ist, und zwar von Agarose und Polysacchariden, mit einem Carbonylierungsreagenz, nämlich 1,1'-Carbonyldiimidazol, gehört zum Stand der Technik; vgl. J. Chromatography, 219 (1981) 353 - 359 und 361 - 372.

Die Anwendungsgebiete für einen erfindungsgemäßen Formgegenstand liegen in der Immunologie, Biochemie, Molekularbiologie, klinischen Diagnostik oder Bioverfahrenstechnik. Beispiele für Einsatzmöglichenkeiten sind lonenaustausch-Chromatographie, Affinitäts-Chromatographie bzw. Affinitätsanreicherung, hydrophobe Chromatographie, Blotting, Dot-Blotting und chemische Festphasensynthese.

Der erfindungsgemäße Formgegenstand kann dadurch herstellbar sein, daß man von einem geformten Polymermaterial auf Zellulosebasis, insbesondere auf Papierbasis, oder von einem geformten Polymermaterial auf Kunststoffbasis ausgeht, insbesondere auf Basis von hydroxyliertem Polyolefin.

Wenn man von einem geformten Polymermaterial auf Zellulosebasis ausgeht, so kann eine breite Palette an Zellulosematerialien mit unterschiedlichsten chemischen Gruppen hergestellt werden. Bei dieser Variante sind die erfindungsgemäßen Formgegenstände durch sehr milde Reaktionsbedingungen, die Verwendung wenig giftiger Materialien und Lösungsmittel sowie einfache technische Durchführung herstellbar. Besonders wichtig is hierbei, daß das underivatisierte Zelluloseausgangsmaterial in seinen chemischen und mechanischen Eigenschaften nur unwesentlich verändert wird. Die chemische Verankerung weist eine ausgezeichnete pH-Stabilität auf, etwa im Bereich von 2 - 12 bei Raumtemperatur.

Der crfindungsgemäße Formgegenstand kann dadurch herstellbar sein, daß man ein Carbonylierungsreagenz
(i) aus der durch 1,1'-Carbonyldiimidazol (CDI), 1,1-Carbonyldi-1.2.4-triazol (CDT), 1,1'-Carbonyldi-1.2.3-benzotriazol (CDB) und/oder Phenoxycarbonyltetrazol (PCT) gebildeten Gruppe und/oder
(ii) aus der durch Chlorameisensäurephenylester (CAP) und/oder Chlorameisensäure-4-nitrophenylester (CANP) gebildeten Gruppe verwendet.

Wenn man mit einem Carbonylierungsreagenz aus Gruppe (i) arbeitet, kann man die Carbonylierung und/oder Umsetzung gemäß Verfahrensstüfe 1(b) in Dimethylformamid (DMF) durchführen, insbesondere bei Raumtemperatur und/oder ohne Pufferzustatz und/oder ohne Zusatz einer weiteren Base neben Dimethylformamid (DMF).

Wenn man mit einem Carbonylierungsreagenz der Gruppe (ii) arbeitet, kann man die Carbonylierung in Pyridin durchführen.

Gemäß einer weiteren Ausführungsform wird die der Erfindung zugrundeliegende Aufgabe durch einen Formgegenstand gelöst, dessen Oberfläche für chemische, biochemische oder biologische Anwendungen mit reaktiven Funktionen versehen ist und der dadurch herstellbar ist, daß man
(a) von einem geformten Polymermaterial als Formgegenstand ausgeht, dessen Oberfläche mit Hydroxylgruppen versehen ist, und den Formgegenstand mit einem Isocyanat und/oder Isothiocyanat umsetzt und Nitrogruppen oder geschützte Aminogruppen einführt und
(b) gegebenenfalls die unumgesetzten Hydroxylgruppen des aktivierten Formgegenstandes blockiert,
(c) gegebenenfalls, sofern bei Stufe (a) durch das Isocyanat und/oder Isothiocyanat Nitrogruppen eingeführt worden sind, diese Nitrogruppen zu Aminogruppen reduziert und
(d) gegebenenfalls, sofern bei Stufe (a) durch das Isocyanat. und/oder Isothiocyanat geschützte Aminogruppen eingeführt worden sind, diese geschützten Aminogruppen zu freien Aminogruppen entschützt.

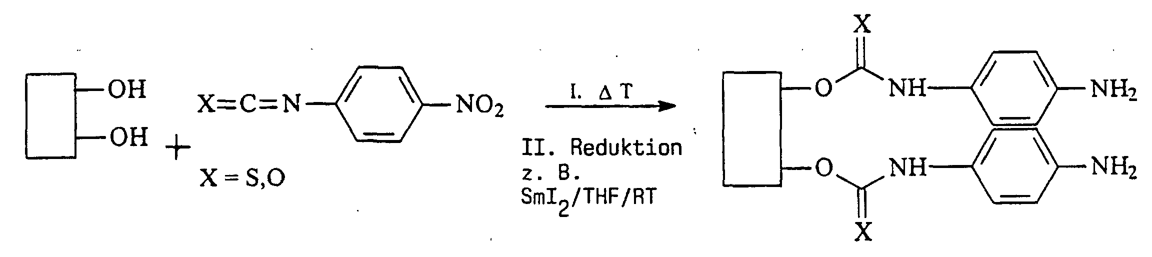

Der erfindungsgemäße Formgegenstand kann dadurch herstellbar sein, daß man von einem geformten Polymermaterial auf Zellulosebasis, insbesondere auf Papierbasis, oder von einem geformten Polymermaterial auf Kunststoffbasis ausgeht, insbesondere auf Basis von hydroxyliertem Polyolefin. Für einen Formgegenstand auf Zellulosebasis kann auf die vorstehenden Ausführungen verwiesen werden.

Der erfindungsgemäße Formgegenstand der zweiten Ausführungsform kann dadurch herstellbar sein, daß man bei Stufe (a) 4-Nitrophenyl-isocyanat oder 4-Nitrophenyl-isothiocyanat verwendet.

Er kann ferner dadurch herstellbar sein, daß man bei Stufe (b) mit Essigsäureanhydrid blockiert.

Er kann ferner dadurch herstellbar sein, daß man bei Stufe (c) mit Samarium-II-iodid reduziert.

Erfindungsgemäß ist der Formgegenstand aus geformtem Polymermaterial dadurch erhältlich, dass man die Umsetzung der Maßnahme (a) und/oder (b) und/oder (c) in Dimethylformamid (DMF) durchführt, insbesondere bei Raumtemperatur und/oder ohne Pufferzusatz und/oder ohne Zusatz einer weiteren Base neben Dimethylformamid (DMF).

Erfindungsgemäße Formgegenstände können dadurch herstellbar sein, dass man von einem geformten Polymermaterial aus der durch Membranen, Folien, miniaturisierten Reaktionsgefäßen, Pipettenspitzen und Mikrotiterplatten gebildeten Gruppe ausgeht.

Nachstehend wird die Erfindung durch Beispiele näher erläutert.

### Beispiele 1 bis 6

### I. Aktivierung

Der im Hochvakuum getrocknete/gefriergetrocknete Formgegenstand (hier: Zelluloseblätter) wird in einem gut verschließbaren, dem Formgegenstand angepaßten Gefäß mit 3 ml/l g Polymer (hier: Zellulose) einer 0,3-molaren. Lösung eines der folgenden Carbonylierungsreagenzien versetzt und bei Raumtemperatur (unter Feuchtigkeitsausschluß) für 6 bis 16 h bewegt. Die Carbonylierungsreagenzien sind:
(i) in absolutem, aminfreiem Dimethylformamid (DMF)
   Beispiel 1: CDI = 1,1'-Carbonyldiimidazol
   Beispiel 2: CDT = 1,1'-Carbonyldi-1,2,4-triazol
   Beispiel 3: CDB = 1,1'-Carbonyldi-1,2,3-benzotriazol
   Beispiel 4: PCT = Phenoxycarbonyltetrazol
(ii) in absolutem Pyridin
   Beispiel 5: CAP = Chlorameisensäurephenylester
   Beispiel 6: CANP = Chlorameisensäure-4-nitrophenylester

### II. Modifizierung

Die Reaktionslösung des Aktivierungsschrittes wird durch Filtration oder Abdekantieren entfernt und der aktivierte Formgegenstand dreimal mit der Hälfte des Volumens des Lösungsmittels der Reaktionslösung für jeweils 5 bis 10 min gespült. Die Waschphasen werden verworfen. 3 ml/l g Polymer einer 0,3-molaren Lösung von Ethylendiamin oder 1,6-Bis-(methylamino)-hexan oder O,O'-Bis-(2-aminopropyl)-polyethylenglycol in DMF oder Pyridin werden zugegeben und bei Raumtemperatur unter Feuchtigkeitsausschluß für wiederum 6 bis 16 h bewegt. Die Reaktionslösung wird dann durch Filtration oder Abdekantieren entfernt und der modifizierte Formgegenstand dreimal mit der Hälfte des Volumens des Lösungsmittels der Reaktionslösung für jeweils 5 bis 10 min gespült. Anschließend wird diese Waschprozedur mit destilliertem Wasser, Ethanol, Aceton, Essigester und Diethylether wiederholt. Nach dem Abziehen des Restethers wird der Formgegenstand im Hochvakuum getrocknet.

### III. Chemische Stabilität

Von den derivatisierten Zelluloseblättern werden 1 cm² große Quadrate folgenden Bedingungen unterworfen und nach 24 Stunden erneut auf ihre Modifizierungsgrade untersucht.
pH 0, 7, 8, 9, 10, 11, 12, 13 und 14 (entsprechend verdünnte NaOH) 24 h/Raumtemperatur;
Konzentrierter wässriger Ammoniak (25 %), 24 h/Raumtemperatur; Trifluoressigsäure (99 %), 24 h/Raumtemperatur;
In allen Fällen konnten die anfänglichen Modifizierungsgrade verifiziert werden.

### Beispiele 7 bis 8

### I. Additionsreaktion

Der im Hochvakuum getrocknete/gefriergetrocknete Formgegenstand (hier: Zelluloseblätter) wird in einem gut verschließbaren, dem Formgegenstand angepaßten Gefäß mit 3 ml einer 0,1-molaren Lösung Isocyanat pro 1 g Polymer (hier: Zellulose) und bei 60 °C (unter Feuchtigkeitsausschluß) im Trockenschrank für 12 h erhitzt.
Beispiel 7: Addition von 4-Nitrophenyl-isocyanat (C₇H₄N₂O₃)[164.12]
Beispiel 8: Addition von 4-Phenyl-isothiocyanat (C₇H₄N₂O₂S)[180.19]
Das Gefäß wird jeweils mehrmals geschwenkt. Die Reaktionslösung des Aktivierungsschrittes wird durch Filtration oder Abdekantieren entfernt und der aktivierte Formgegenstand mit jeweils 3 ml Lösungsmitteln pro g Trockengewicht des eingesetzten Polymers für jeweils 5 bis 10 min geschwenkt. Die Waschphasen werden nach dem Abdekantiercn oder Abfiltrieren verworfen.

Die Lösungsmittel werden in der folgenden Reihenfolge eingesetzt: Dimethylformamid (pA), Wasser (bidest.) Ethanol (zS), Aceton (zS), Essigsäureethylester (zS), Dichlormethan (pA). Nach Abdampfen des organischen Lösungsmittels wird der aktivierte Formgegenstand im Hochvakuum bei Raumtemperatur getrocknet.

### II. Capping (Blockierung von bei Stufe I nicht umgesetzten Funktionen der Polymeroberfläche)

Der vorgetrocknete aktivierte Formgegenstand wird in 3 ml DMF/Pyridin (2:1, Volumen/Volumen) pro g Trockengewicht vorgelegt und anschließend mit 0,5 ml Essigsäureanhydrid versetzt. Nach 6 h bei Raumtemperatur und moderatem Schwenken wird die Reaktion abgebrochen. Die Reaktionslösung wird abdekantiert oder abfiltriert und die Waschprozedur wie bei Stufe I wiederholt.

### III. Reduktion der Nitrogruppe mit Samarium-II-iodid

Der vorgetrocknete aktivierte Formgegenstand wird in 10 ml DMF (abs) pro g Trockengewicht vorgelegt und mit 2 ml einer 0,1-molaren Lösung Samarium-II-iodid in THF (abs) pro g Trockengewicht versetzt. Man schwenkt das Reaktionsgefäß unter Feuchtigkeitsausschluß für 10 bis 16 h bei Raumtemperatur. Nach Abdekantieren der Reaktionslösung wird der Formgegenstand mit 5 ml DMF/g Trockengewicht für 15 min und anschließend zweimal mit 10 ml Wasser (bidest.) für ca. 2 bis 3 h geschwenkt. Daraufhin werden die Waschprozeduren gemäß Stufe 1 wiederholt.

## Patentansprüche

1. Formgegenstand, dessen Oberfläche für chemische, biochemische oder biologische Anwendungen mit reaktiven Funktionen versehen ist, dadurch herstellbar, daß man
(a) von einem geformten Polymermaterial auf Papierbasis als Formgegenstand ausgeht, dessen Oberfläche mit Hydroxylgruppen versehen ist, und den Formgegenstand in an sich bekannter Weise mit einem Carbonylierungsreagenz umsetzt und
(b) den carbonylierten Formgegenstand mit einer Verbindung der Formel R₃-NH-R₄, worin R₃ den Rest eines an sich bekannten primären Amins und R₄ ein H-Atom oder R₃ und R₄, die gleich oder verschieden sein können, Reste eines an sich bekannten sekundären Amins darstellen, zu einem Urethan-Derivat umsetzt.

2. Formgegenstand nach Anspruch 1, dadurch herstellbar, daß man ein Carbonylierungsreagenz (i) aus der durch 1,1'-Carbonyldiimidazol (CDI), 1,1-Carbonyldi-1.2.4-triazol (CDT), 1,1'-Carbonyldi-1.2.3-benzotriazol (CDB) und/oder Phenoxycarbonyltetrazol (PCT) gebildeten Gruppe und/oder (ii) aus der durch Chlorameisensäurephenylester (CAP) und/oder Chlorameisensäure-4-nitrophenylester (CANP) gebildeten Gruppe verwendet.

3. Formgegenstand nach Anspruch 2 (i), dadurch herstellbar, daß man die Carbonylierung und/oder Umsetzung gemäß Anspruch 1(b) in Dimethylformamid (DMF) durchführt, insbesondere bei Raumtemperatur und/oder ohne Pufferzusatz und/oder ohne Zusatz einer weiteren Base neben Dimethylformamid (DMF).

4. Formgegenstand nach Anspruch 2 (ii), dadurch herstellbar, daß man die Carbonylierung in Pyridin durchführt.

5. Formgegenstand, dessen Oberfläche für chemische, biochemische oder biologische Anwendungen mit reaktiven Funktionen versehen ist, dadurch herstellbar, daß man
(a) von einem geformten Polymermaterial als Formgegenstand ausgeht, dessen Oberfläche mit Hydroxylgruppen versehen ist, und den Formgegenstand mit einem Isocyanat und/oder Isothiocyanat umsetzt und Nitrogruppen oder geschützte Aminogruppen einführt und
(b) gegebenenfalls die unumgesetzten Hydroxylgruppen des aktivierten Formgegenstandes blockiert,
(c) gegebenenfalls, sofern bei Stufe (a) durch das Isocyanat und/oder Isothiocyanat Nitrogruppen eingeführt worden sind, diese Nitrogruppen zu Aminogruppen reduziert und
(d) gegebenenfalls, sofern bei Stufe (a) durch das Isocyanat und/oder Isothiocyanat geschützte Aminogruppen eingeführt worden sind, diese geschützten Aminogruppen zu freien Aminogruppen, entschützt.

6. Formgegenstand nach Anspruch 5, dadurch herstellbar, daß man von einem geformten Polymermaterial auf Zellulosebasis ausgeht, insbesondere auf Papierbasis.

7. Formgegenstand nach Anspruch 5, **dadurch gekennzeichnet, daß** man von einem geformten Polymermaterial auf Kunststoffbasis ausgeht, insbesondere auf Basis von hydroxyliertem Polyolefin.

8. Formgegenstand nach Anspruch 5, 6 oder 7, dadurch herstellbar, daß man bei Stufe (a) 4-Nitrophenyl-isocyanat oder 4-Nitrophenyl-iso-thiocyanat verwendet.

9. Formgegenstand nach einem der Ansprüche 5 bis 8, dadurch herstellbar, daß man bei Stufe (b) mit Essigsäureanhydrid blokkiert.

10. Formgegenstand nach einem der Ansprüche 5 bis 9, dadurch herstellbar, daß man bei Stufe (c) mit Samarium-II-iodid reduziert.

11. Formgegenstand nach einem der Ansprüche 5 bis 10, dadurch herstellbar, daß man die Umsetzung gemäß Anspruch 5(a) und/oder 5(b) und/oder 5(c) in Dimethylformamid (DMF) durchführt, insbesondere bei Raumtemperatur und/oder ohne Pufferzusatz und/oder ohne Zusatz einer weiteren Base neben Dimethylformamid (DMF).

12. Formgegenstand nach einem der vorhergehenden Ansprüche, dadurch herstellbar, daß man von einem geformten Polymermaterial aus der durch Membranen, Folien, miniaturisierte Reaktionsgefäße, Pipettenspitzen und Mikrotiterplatten gebildeten Gruppe ausgeht.

## Claims

1. Shaped article the surface of which is provided with reactive functions for chemical, biochemical or biological applications, which can be manufactured by
(a) starting from, as the shaped article, a shaped paper-based polymeric material, the surface of which shaped article is provided with hydroxy groups, and reacting the shaped article with a carbonylating reagent in a manner known *per se*, and
(b) reacting the carbonylated shaped article with a compound of the formula R₃-NH-R₄, wherein R₃ represents the radical of a primary amine known *per se* and R₄ represents a hydrogen atom or R₃ and R₄, which may be the same or different, represent radicals of a primary amine known *per se,* to form a urethane derivative.

2. Shaped article according to claim 1, which can be manufactured by using a carbonylating reagent (i) from the group consisting of 1,1'-carbonyldiimidazole (CDI), 1,1-carbonyldi-1,2,4-triazole (CDT), 1,1'-carbonyldi-1,2,3-benzotriazole (CDB) and/or phenoxycarbonyltetrazole (PCT) and/or (ii) from the group consisting of chloroformic acid phenyl ester (CFP) and/or chloroformic acid 4-nitrophenyl ester (CFNP).

3. Shaped article according to claim 2 (i), which can be manufactured by carrying out the carbonylation and/or reaction according to claim 1(b) in dimethylformamide (DMF), especially at room temperature and/or without addition of a buffer and/or without addition of a further base besides dimethylformamide (DMF).

4. Shaped article according to claim 2 (ii), which can be manufactured by carrying out the carbonylation in pyridine.

5. Shaped article the surface of which is provided with reactive functions for chemical, biochemical or biological applications, which can be manufactured by
(a) starting from, as the shaped article, a shaped polymeric material, the surface of which shaped article is provided with hydroxy groups, and reacting the shaped article with an isocyanate and/or isothiocyanate, and introducing nitro groups or protected amino groups, and
(b) optionally blocking the unreacted hydroxy groups of the activated shaped article,
(c) optionally, if nitro groups have been introduced by the isocyanate and/or isothiocyanate in step (a), reducing any such nitro groups to form amino groups, and
(d) optionally, if protected amino groups have been introduced by the isocyanate and/or isothiocyanate in step (a),
de-protecting any such protected amino groups to form free amino groups.

6. Shaped article according to claim 5, which can be manufactured by starting from a shaped cellulose-based polymeric material, especially a paper-based polymeric material.

7. Shaped article according to claim 5, **characterized in that** a shaped plastics-based polymeric material, especially a hydroxylated-polyolefin-based polymeric material, is used as starting material.

8. Shaped article according to claim 5, 6 or 7, which can be manufactured by using 4-nitrophenyl isocyanate or 4-phenyl isothiocyanate in step (a).

9. Shaped article according to any one of claims 5 to 8, which can be manufactured by carrying out the blocking in step (b) with acetic anhydride.

10. Shaped article according to any one of claims 5 to 9, which can be manufactured by carrying out the reduction in step (c) with samarium (II) iodide.

11. Shaped article according to any one of claims 5 to 10, which can be manufactured by carrying out the reaction according to claim 5(a) and/or 5(b) and/or 5(c) in dimethylformamide (DMF), especially at room temperature and/or without addition of a buffer and/or without addition of a further base besides dimethylformamide (DMF).

12. Shaped article according to any one of the preceding claims, which can be manufactured by starting from a shaped polymeric material from the group consisting of membranes, films, miniaturized reaction vessels, pipette tips and microtitre plates.

## Revendications

1. Article moulé portant à sa surface des fonctions réactives pour des applications chimiques, biochimiques ou biologiques, que l'on peut préparer
(a) en partant d'un article moulé en un matériau polymère moulé à base de papier, portant à sa surface des groupes hydroxyle, et en faisant réagir l'article moulé d'une manière connue avec un réactif de carbonylation, et
(b) en faisant réagir l'article moulé carbonylé avec un composé de formule R₃-NH-R₄, où R₃ représente le résidu d'une amine primaire connue et R₄ un atome d'hydrogène ou R₃ et R₄, identiques ou différents, représentent chacun le résidu d'une amine secondaire connue, de manière à obtenir un dérivé de type uréthanne.

2. Article moulé selon la revendication 1, que l'on peut préparer en utilisant un réactif de carbonylation (i) choisi parmi le 1,1'-carbonyldiimidazole (CDI), le 1,1-carbonyldi-1.2.4-triazole (CDT), le 1,1'-carbonyldi-1.2.3-benzotriazole (CDB) et/ou le phénoxycarbonyltétrazole (PCT) et/ou (ii) choisi parmi le chloroformiate de phényle (CAP) et/ou le chloroformiate de 4-nitrohényle (CANP).

3. Article moulé selon la revendication 2 (i), que l'on peut préparer en mettant en oeuvre la réaction de carbonylation et/ou la réaction de la revendication 1(b) dans du diméthylformamide (DMF), en particulier à température ambiante et/ou en l'absence d'un agent tampon et/ou sans addition d'une base autre que le diméthylformamide (DMF).

4. Article moulé selon la revendication 2 (ii), que l'on peut préparer en mettant en oeuvre la réaction de carbonylation dans de la pyridine.

5. Article moulé portant à sa surface des fonctions réactives pour des applications chimiques, biochimiques ou biochimiques, que l'on peut préparer
(a) en partant d'un article moulé en un matériau polymère moulé portant à sa surface des groupes hydroxyle, et en faisant réagir l'article moulé avec un isocyanate et/ou un isothiocyanate introduisant ainsi des groupes nitro ou des groupes amino protégés,
(b) en bloquant éventuellement les groupes hydroxyle n'ayant pas réagi de l'article moulé,
(c) en réduisant les groupes nitro, éventuellement introduits dans l'étape (a) par l'isocyanate et/ou l'isothiocyanate, de manière à les convertir en groupes amino, et
(d) en convertissant les groupes amino protégés, éventuellement introduits dans l'étape (a) par l'isocyanate et/ou l'isothiocyanate, en groupes amino libres.

6. Article moulé selon la revendication 5, que l'on peut préparer en partant d'un matériau polymère moulé à base de cellulose, en particulier à bàse de papier.

7. Article moulé selon la revendication 5, **caractérisé par le fait que** l'on part d'un matériau polymère moulé à base de polymère synthétique, en particulier à base de polyoléfines hydroxylées.

8. Article moulé selon la revendication 5, 6 ou 7, que l'on peut préparer en utilisant dans l'étape (a) du 4-nitrophényl-isocyanate ou du 4-nitrophényl-isothiocyanate.

9. Article moulé selon l'une des revendications 5 à 8, que l'on peut préparer en utilisant pour le blocage de l'étape (b) de l'anhydride acétique.

10. Article moulé selon l'une des revendications 5 à 9, que l'on peut préparer en utilisant pour la réduction de l'étape (c) de l'iodure de samarium (II).

11. Article moulé selon une des revendications 5 à 10, que l'on peut préparer en mettant en oeuvre la réaction selon la revendication 5(a) et/ou 5(b) et/ou 5(c) dans du diméthylformamide (DMF), en particulier à température ambiante et/ou en l'absence d'agent tampon et/ou sans addition d'une base autre que le dimétylformamide (DMP).

12. Article moulé selon l'une des revendications précédentes, que l'on peut préparer en partant d'un matériau polymère moulé choisi parmi les membranes, les feuilles, les récipients réactifs miniaturisés, les cônes de pipettes et les plaques de microtitrage.
